Europäisches Patentamt

European Patent Office

Office européen des brevets

(11) Veröffentlichungsnummer: **0 016 899**
A1

(12) **EUROPÄISCHE PATENTANMELDUNG**

(21) Anmeldenummer: 80100067.0

(22) Anmeldetag: 07.01.80

(51) Int. Cl.³: **C 07 C 51/42**, C 07 C 57/145,
C 07 C 51/573, C 07 C 57/15,
C 07 D 307/60, C 07 D 307/88,
C 07 C 55/14, C 07 C 63/26,
C 07 C 67/48, C 07 C 69/44,
C 07 C 69/82

(30) Priorität: 21.03.79 DE 2910973

(71) Anmelder: Krupp-Koppers GmbH, Moltkestrasse 29,
D-4300 Essen 1 (DE)

(43) Veröffentlichungstag der Anmeldung: 15.10.80
Patentblatt 80/21

(72) Erfinder: Coenen, Hubert, Dr.Dipl.-Ing, Wortbergrode 13,
D-4300 Essen 1 (DE)
Erfinder: Kriegel, Ernst, Dr.Dipl.-Ing., Juistweg 51,
D-4300 Essen 1 (DE)
Erfinder: Preusser, Gerhard, Dr.Dipl.-Chem,
Lönsberg 24, D-4300 Essen 1 (DE)
Erfinder: Schmid, Karl, Dr.Dipl.-Chem, Hohe Buchen 14,
D-4300 Essen 1 (DE)
Erfinder: Schulze, Martin, Krüdenscheider Weg 83,
D-5604 Neviges (DE)
Erfinder: Wetzel, Rolf, Dr.Dipl.-Ing, Anemonenweg 22,
D-5628 Heiligenhaus (DE)

(84) Benannte Vertragsstaaten: BE FR GB IT LU NL SE

(54) Verfahren zur Gewinnung von reinen Dicarbonsäuren, deren Anhydriden und/oder Estern.

(57) Verfahren zur Gewinnung von reinen Dicarbonsäuren, deren Anhydriden und/oder Estern (27) aus diese Verbindungen enthaltenden Rohprodukten (12) wobei diese mit Gasen oder Gasgemischen (1), die sich im überkritischen Zustande befinden, bei Temperaturen oberhalb der kritischen Temperatur und Drücken oberhalb des kritischen Druckes des verwendeten Gases bzw. Gasgemisches einer Extraktion (11) unterworfen werden. Dabei können folgende Gase bzw. gasförmige Verbindungen sowohl einzeln als auch in Form von Mischungen untereinander als Extraktionsmittel zur Anwendung gelangen: $CO_2$, $N_2O$, Fluor/Chlor-Kohlenwasserstoffe, $NH_3$, $SO_2$, $CH_3Cl$, $C_2H_4$, $C_2H_6$ und $CH_3OH$. Das Verfahren ist insbesondere zur Reinigung der durch Luftoxidation von Kohlenwasserstoffen gewonnenen Rohprodukte zur Herstellung von Fumarsäure, Maleinsäure, Phthalsäureanhydrid, Adipinsäure, Adipinsäuredimethylester, Terephthalsäure und Terephthalsäuredimethylester geeignet.

Essen, den 7. März 1979
N 4778/7 d    Dr.Ha/Wi.

0016899

KRUPP-KOPPERS GMBH, Moltkestrasse 29, 4300 Essen

Verfahren zur Gewinnung von reinen Dicarbonsäuren,
deren Anhydriden und/oder Estern.

Die Erfindung betrifft ein Verfahren zur Gewinnung von reinen Dicarbonsäuren, deren Anhydriden und/oder Estern aus diese Verbindungen enthaltenden Rohprodukten.

Es ist bekannt, dass viele zu den vorstehend genannten Stoffklassen gehörende Verbindungen wichtige Grundchemikalien für die Herstellung von Kunststoffen bzw. Kunstfasern darstellen. Da jedoch bereits geringe Verunreinigungen in diesen Grundchemikalien zu erheblichen Qualitätsverschlechterungen der daraus hergestellten Produkte führen, wie z.B. mangelnde Farbstabilität und verminderte mechanische Festigkeit, ist man gezwungen, an die Reinheit der verwendeten Grundchemikalien besonders hohe Anforderungen zu stellen. Dies ist insbesondere notwendig, wenn diese Verbindungen durch - zumeist katalytische - Oxidation von Kohlenwasserstoffen mit Luft oder Sauerstoff in der Gas- oder Flüssigphase im grosstechnischen Massstab erzeugt werden. Dabei können Zwischen- oder Nebenprodukte auftreten, die zu erheblichen Verunreinigungen des bei der Oxidation gewonnenen Rohproduktes führen.

./.

So konnten z. B. im Roh-Oxidat der Gasphasenoxidation von Benzol zu Maleinsäureanhydrid Chinon, Hydrochinon, Phenol und auch Diphenol nachgewiesen werden. Bei der katalytischen Gasphasenoxidation von Naphthalin oder o-Xylol zu Phthalsäureanhydrid können - je nach Ausgangsprodukt - im Rohoxidat o-Toluylaldehyd, o-Toluylsäure, Phthalid und Naphthochinon als kritische Verunreinigungen auftreten. Die besonders störenden Verunreinigungskomponenten bei der in Flüssigphase durchgeführten direkten Oxidation des p-Xylols zu Roh-Terephthalsäure (TPS) sind p-Toluylsäure (TS), Terephthalaldehydsäure (TAS) und Benzoesäure (BS) und im Falle der Oxidation über die Dimethylterephthalat (DMT)-Route die Methanolester dieser Verbindungen. Dabei muss besonderer Wert auf möglichst weitgehende Entfernung dieser Verunreinigungen gelegt werden, weil sie bei der weiteren Kondensation mit Glykolen zu Polyalkylenterephthalaten zu Kettenabbrüchen mit der Folge verminderter mechanischer Faserfestigkeit und Farbstabilität führen. So soll z. B. der Gehalt an TAS, die in der Roh-TPS mit einem Gehalt von 1,0 - 2,0 % vorliegt, in der Rein-TPS einen Wert von 25 ppm nicht überschreiten.

Zur Entfernung dieser Verunreinigungen sind eine Anzahl von Verfahren vorgeschlagen worden, von denen ein Teil auch bei der industriellen Herstellung von Dicarbonsäuren und deren Derivaten angewandt wird.

0016899
7. 3. 1979
N 4778/7 d

Beispielsweise werden die Rohprodukte der Maleinsäure- und Phthalsäureanhydrid-Produktion zumeist zunächst einer längeren Wärmebehandlung bei 150 - 180°C - gegebenenfalls unter Zusatz von katalytisch wirkenden Chemikalien - unterzogen und anschliessend durch Destillationsprozesse gereinigt. Bei der Wärmebehandlung wird ein Teil der Verunreinigungskomponenten durch unkontrollierte Polykondensation in teerähnliche Produkte übergeführt, die dann im nachfolgenden Destillationsprozess entfernt werden müssen, wobei lästige Betriebserschwernisse durch die Verschmutzung von Wärmetauscherflächen und auch Ausbeuteverluste auftreten.

Zur Reinigung von Roh-DMT zu faserreinem Produkt ist eine Kombination von Destillations- und Kristallisationsprozessen erforderlich, die ebenfalls sicher kein wirtschaftliches Optimum darstellen.

Besonders kritisch ist die Reinigung der bei der direkten Oxidation von p-Xylol anfallenden Roh-TPS. TPS weist eine Sublimationstemperatur von 402°C (im geschlossenen Rohr) auf und ist in gebräuchlichen organischen Lösungsmitteln praktisch unlöslich. Auch in Wasser ist die Löslichkeit bis 100°C sehr gering, so dass wegen dieser für Reinigungsoperationen sehr nachteiligen Eigenschaften TPS auch als "organischer Sand" bezeichnet wird. Es sind daher in jüngerer Zeit auch andere Wege zur Reinigung von Roh-TPS auf Faserqualität vorgeschlagen worden, die

./.

- 4 -

jedoch gleichfalls mit einem hohen wirtschaftlichen Aufwand verbunden sind. Neben Umkristallisationsprozessen bei sehr hohen Drücken und Temperaturen, bei denen auch unkontrollierbare chemische Umsetzungen stattfinden, wurden sogar gesonderte katalytische Prozesse - z.B. zur Überführung der TAS in Benzoesäure (BS) unter Abspaltung von CO - vorgeschlagen und teilweise technisch ausgeführt.

Die vorstehenden Ausführungen lassen erkennen, dass in der Fachwelt ein echtes Bedürfnis dafür vorlag, die Gewinnung von reinen Dicarbonsäuren, deren Anhydriden und/oder Estern aus diese Verbindungen enthaltenden Rohprodukten zu vereinfachen und zu verbessern, wobei zur Vermeidung von Zersetzungsreaktionen insbesondere auf milde Temperaturbedingungen geachtet werden soll.

Das der Lösung dieser Aufgabe dienende Verfahren ist erfindungsgemäss dadurch gekennzeichnet, dass die Rohprodukte mit Gasen oder Gasgemischen, die sich im überkritischen Zustande befinden, bei Temperaturen oberhalb der kritischen Temperatur und Drücken oberhalb des kritischen Druckes des verwendeten Gases bzw. Gasgemisches einer Extraktion unterworfen werden.

Die erfindungsgemässe Arbeitsweise sieht also vor, dass das als Einsatzgut dienende Rohprodukt zum Zwecke der Reinigung einer Extraktion mit

./.

Gasen unterworfen wird, die sich hinsichtlich ihrer Temperatur und hinsichtlich ihres Druckes im überkritischen Zustande befinden. Prinzipiell ist die Extraktion mit überktitischen Gasen zwar bereits seit einiger Zeit bekannt. Bisher wurde diese Methode jedoch vor allem zur Gewinnung von Naturstoffen eingesetzt. Ihre Anwendung für den erfindungsgemässen Zweck eröffnet im Hinblick auf die besonderen Eigenschaften der überkritischen Gase als Lösungsmittel neue Perspektiven für die Reinigung der Dicarbonsäuren und deren Derivaten unter milden thermischen Bedingungen. Überkritische Gase können eine starke Zunahme der Löslichkeit für bestimmte Komponenten bis zu einem Faktor 10 000 aufweisen, wenn der kritische Zustand überschritten wird. Damit werden hohe Transportraten bei geringen Temperaturänderungen in der Gasphase möglich.

Die erfindungsgemässe Extraktion mit überkritischen Gasen wird üblicherweise aus wirtschaftlichen Gründen bei Temperaturen und Drücken durchgeführt, die nur geringefügig oberhalb der kritischen Temperatur- und Druckwerte des angewandten Extraktionsmittels liegen, um die Energiekosten niedrig zu halten. Dabei hat sich/herausgestellt, dass die allerdings Transportraten am höchsten sind, wenn die Temperatur dicht oberhalb der kritischen Temperatur, die Drücke aber erheblich oberhalb der kritischen Drücke eingestellt werden. Als gasförmige Extraktionsmittel kommen z.B. in Betracht :

./.

0016899
7. 3. 1979
N 4778/7 d

|  | $T_{krit.}$ | $P_{krit.}$ |
|---|---|---|
| $CO_2$ | 31,0 °C | 73,9 bar |
| $N_2O$ | 36,5 °C | 72,8 bar |
| $C_2H_6$ | 32,2 °C | 49,9 bar |
| $C_2H_4$ | 10,0 °C | 51,2 bar |
| $CClF_3$ | 28,8 °C | 38,6 bar |

und ähnliche Chlor/Fluor-Kohlenwasserstoffe.

Für die Anwendung höherer Extraktionstemperaturen kommen auch folgende Gase/als Extraktionsmittel in Frage: bzw. gasförmigen Verbindungen

|  | $T_{krit.}$ | $P_{krit.}$ |
|---|---|---|
| $SO_2$ | 157,5 °C | 78,8 bar |
| $NH_3$ | 132,4 °C | 113 bar |
| $CH_3Cl$ | 143 °C | 66 bar |
| $CH_3OH$ | 240 °C | 99 bar |

Ein besonders bevorzugtes Extraktionsmittel ist $CO_2$. Es besitzt eine hervorragende Selektivität und eine ausreichende Löslichkeit für die zu extrahierenden Komponenten, ist physiologisch unbedenklich, preisgünstig und leicht verfügbar.

Die genannten Extraktionsmittel können dabei sowohl einzeln als auch in Form von Mischungen untereinander zur Anwendung gelangen.

Das erfindungsgemässe Verfahren eignet sich sowohl zur Gewinnung reiner aliphatischer als auch reiner aromatischer Dicarbonsäuren, deren Anhydride und/oder Ester aus den entsprechenden Rohprodukten. Als Beispiele werden - ohne die Erfindung damit einschränken zu wollen - genannt: Fumarsäure, Maleinsäure, Maleinsäureanhydrid, Adipinsäure und deren Ester, Phthalsäure, Phthalsäureanhydrid, Trimellithsäure, Trimellithsäureanhydrid, Terephthalsäure und Terephthalsäuredimethylester.

Besonders vorteilhaft ist die Anwendung des erfindungsgemässen Verfahrens, wenn das als Einsatzgut dienende Rohprodukt durch Oxidation von Kohlenwasserstoffen in der Gas- oder Flüssigkeitsphase erzeugt wurde. Das als Einsatzgut dienende Rohprodukt kann so wie es anfällt oder in Form einer Suspension bzw. Lösung in hierfür geeigneten flüssigen Suspensions- oder Lösungsmitteln der Extraktion unterworfen werden. Es kann aber auch in Form einer Schmelze in den Extraktor eingeleitet werden.

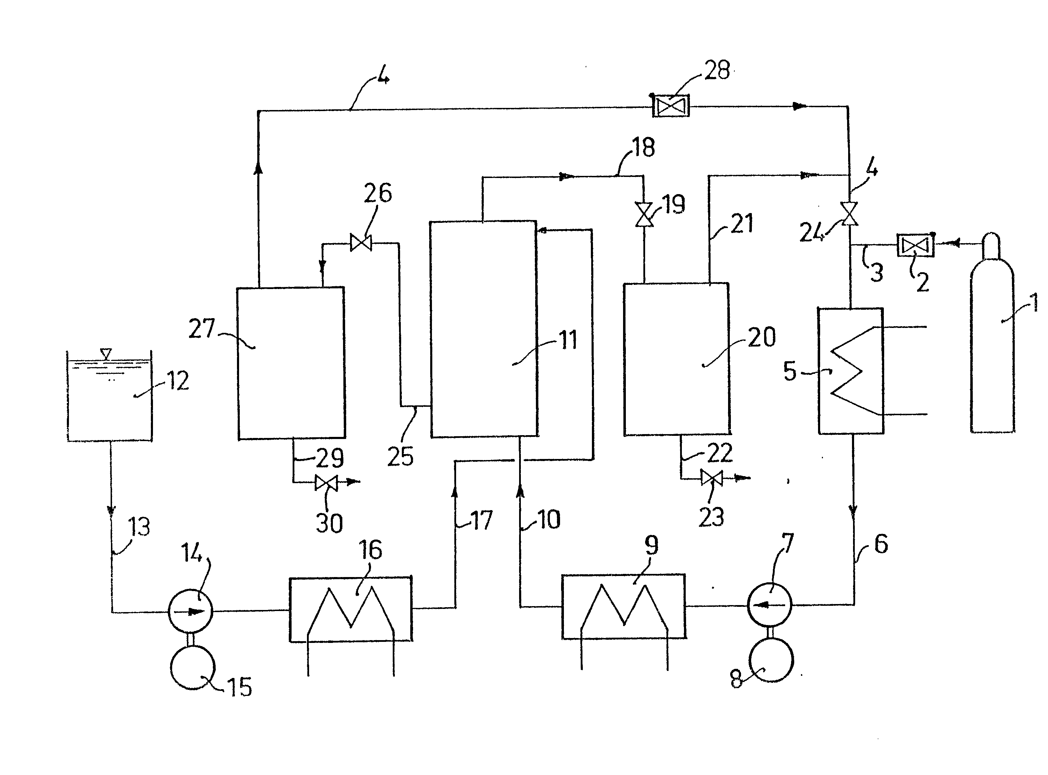

Weitere Einzelheiten des erfindungsgemässen Verfahrens und der zu seiner Durchführung dienenden Vorrichtung sollen nachfolgend an Hand des in der Abbildung dargestellten Fliessschemas erläutert werden.

Das als Extraktionsmittel dienende Gas bzw. Gasgemisch gelangt dabei

./.

0016899
7. 3. 1979
N 4778/7 d

aus dem Gasbehälter 1 über das Regulierventil 2 und die Leitung 3 in die Leitung 4, die zum indirekten Kühler 5 führt. Hier wird das Gas so abgekühlt, dass es diesen Kühler über die Leitung 6 im verflüssigten Zustande verlässt. Die in dieser Leitung befindliche Flüssigkeitspumpe 7 mit dem Manometer 8 dient zur Umwälzung und erforderlichen Druckerhöhung des Gases, das anschliessend in den Wärmeaustauscher 9 eintritt, in dem die erforderliche Temperaturerhöhung erfolgt. Nachdem das Gas durch entsprechende Druck- und Temperaturerhöhung in den überkritischen Zustand gebracht worden ist, gelangt es über die Leitung 10 von unten in den als Extraktor dienenden Autoklaven 11.

Gleichzeitig wird das im Vorratsbehälter 12 befindliche, als Einsatzgut dienende Rohprodukt, das meistens im festen Zustande vorliegt, über die Leitung 13 abgezogen. Die in dieser Leitung befindliche Förderpumpe 14 mit dem Manometer 15 dient der notwendigen Förderung und Verdichtung des Rohproduktes, das nunmehr in den Wärmeaustauscher 16 gelangt, in dem es die notwendige Aufwärmung erfährt, so dass es anschliessend mit einer Temperatur, die oberhalb der kritischen Temperatur und mit einem Druck, der oberhalb des kritischen Druckes des als Extraktionsmittel verwendeten Gases bzw. Gasgemisches liegt, über die Leitung 17 in den Oberteil des Autoklaven 11 eingeleitet werden kann.

./.

Bei der in der Abbildung dargestellten kontinuierlichen Betriebsweise wird das Rohprodukt von oben nach unten und das als Extraktionsmittel dienende Gas im Gegenstrom von unten nach oben durch den Autoklaven 11 geleitet. Das im überkritischen Zustande befindliche Gas durchströmt auf Grund seiner geringeren Dichte das Rohprodukt und belädt sich entsprechend dem thermodynamischen Zustand mit einer oder mehreren Komponenten des Rohproduktes, die vorwiegend als Verunreinigungen anzusehen sind. Der Autoklav 11 kann die Konstruktionsmerkmale bekannter Extraktoren aufweisen. Insbesondere kann er mit Einbauten zum verbesserten Stoffaustausch, wie z.B. Siebböden oder dergl., versehen sein. Voraussetzung ist natürlich, die Konstruktion des Autoklaven 11 besitzt die erforderliche Druckfestigkeit.

Es ist aber gegebenenfalls natürlich auch möglich, dass das Rohprodukt (Einsatzgut) und das Gas im Gleich- oder Kreuzstrom zueinander geführt werden, wobei ersteres vor allem bei diskontinuierlicher Arbeitsweise angewandt wird.

Die beladene Gasphase (Extrakt) wird aus dem Oberteil des Autoklaven 11 durch die Leitung 18 abgezogen und anschliessend vor dem Eintritt in den Abscheider 20 durch das Drosselventil 19 entspannt. Infolge der Druckerniedrigung und Abkühlung verliert das Gas, entsprechend seinem neuen thermodynamischen Zustand, ganz oder teilweise seine Aufnahmefähigkeit für organische Stoffe. Eine Abscheidung der aufgenommenen Komponenten findet aber auch bei Druckerniedrigung und Temperaturerhöhung oder bei Temperaturerhöhung statt. Dabei werden die im Autoklaven 11 aufgenommenen Komponenten, die im wesentlichen die Verunreinigungen des Rohproduktes darstellen, teilweise oder vollständig abgeschieden und sammeln sich am Boden des Abscheiders 20. Von hier aus können sie über die Leitung 22 und das Ventil 23 teils in flüssiger, teils in fester Form abgezogen werden. Gegebenenfalls können diese Komponenten in die der Herstellung des Rohproduktes dienende Verfahrensstufe (Luftoxidation von Kohlenwasserstoff) oder eine andere vorgeschaltete Verfahrensstufe zurückgeführt werden. Das entspannte und regenerierte Extraktionsmittel verlässt im gasförmigen Zustande den Abscheider 20 über die Leitung 21 und wird nach Passieren des Ventils 24 in den indirekten Kühler 5 zurückgeführt, so dass es danach im verflüssigten Zustande erneut in den weiter oben beschriebenen Extraktionskreislauf gelangt.

Anstelle der im vorliegenden Falle vorgesehenen Arbeitsweise, bei der das als Extraktionsmittel dienende Gas zunächst verflüssigt und danach durch Druckerhöhung in den überkritischen Zustand überführt wird, ist es auch möglich, das Extraktionsmittel vom gasförmigen Zustande durch Druckerhöhung direkt in den überkritischen Zustand zu überführen. Bei dieser Verfahrensvariante kann dann der indirekte Kühler 5 in Fortfall kommen.

./.

· 0016899
7. 3. 1979
N 4778/7 d

Im Unterteil bzw. am Boden des Autoklaven 11 wird währenddessen das von den Verunreinigungen befreite Reinprodukt mit dem gelösten Extraktionsmittel als Raffinatphase über die Leitung 25 abgezogen und im Drosselventil 26 entspannt. In dem nachgeschalteten Abscheider 27 sammelt sich das Raffinat am Boden und kann über die Leitung 29 und das Ventil 30 als Reinprodukt aus dem Prozess entfernt werden, während das Extraktionsmittel am Kopf des Abscheiders 27 abgezogen und über die Leitung 4, das Rückschlagventil 28 sowie das Ventil 24 in den weiter oben beschriebenen Extraktionskreislauf zurückgeführt wird.

Im Gegensatz zu der vorstehend beschriebenen kontinuierlichen Arbeitsweise kann der Autoklav 11 auch diskontinuierlich, d.h. chargenweise, mit dem zu reinigenden Rohprodukt gefüllt werden. Der Extraktionsmittelkreislauf bewirkt wieder eine Verarmung des Rohproduktes an den zu entfernenden Verunreinigungen, die sich beim Entspannen der Extraktphase im Abscheider 20 ansammeln. Das Raffinat wird nach einer bestimmten Umwälzzeit des Extraktionsmittels (Extraktionszeit) aus dem Autoklaven 11 entnommen und in der weiter oben beschriebenen Art und Weise über den Abscheider 27 aus dem Prozess abgezogen. Der Chargenbetrieb ist dabei insbesondere geeignet für den Einsatz von Feststoffen. Selbstverständlich kann die Extraktion gegebenenfalls auch mehrstufig ausgeführt werden, wobei zwei oder mehrere Autoklaven hintereinander angeordnet sind.

./.

Abschliessend soll die Wirksamkeit des erfindungsgemässen Verfahrens durch die beiden nachfolgenden Verfahrensbeispiele belegt werden. Das erste Beispiel betrifft die Gewinnung von reinem Phthalsäureanhydrid und das zweite Beispiel die Gewinnung von reiner Terephthalsäure.

**Beispiel 1 :**

Als Einsatzgut wurde ein Roh-Oxidat aus einer grosstechnischen Anlage zur Herstellung von Phthalsäureanhydrid durch Oxidation von o-Xylol mit Luft am Festbett-Katalysator benutzt.

500 g geschmolzenes, braun verfärbtes Roh-Oxidat wurden in einem mit Sprudeleinrichtung versehenen Extraktionsautoklaven eingefüllt. Über einen Zeitraum von 50 Min. wurde bei 150° C und 120 bar $CO_2$ über die Sprudeleinrichtung durch das geschmolzene Roh-Oxidat geleitet. Anschliessend wurde das $CO_2$ in einem Abscheidegefäss auf unterkritische Bedingungen entspannt und das extrahierte Stoffgemisch abgeschieden.

Im Reaktionsautoklaven verblieben    445 g

im Abscheidegefäss verblieben    27 g

Verlust (in den Rohrleitungen)    28 g

./.

Von dem im Extraktionsautoklaven bzw. im nachgeschalteten Reinprodukt-Abscheider verbliebenen, jetzt reinweissen Phthalsäureanhydrid wurde die Hazenzahl nach ASTM D 2280-64 und der Erstarrungspunkt nach ASTM D 1463-64 bestimmt.

| | |
|---|---|
| Erstarrungspunkt | 130,9 ° C |
| Hazenzahl | 10 |

Bei dem Versuch wurde somit ein den üblichen Reinheitsanforderungen
entsprechendes Reinprodukt mit einer Ausbeute von 89 % bezogen auf
eingesetztes Roh-Oxidat gewonnen.

Beispiel 2 :

Das Einsatzgut Rohterephalsäure aus einer technischen Flüssigphase-
Oxidation hatte folgende Zusammensetzung :

| | | |
|---|---|---|
| TPS | 97,6 Gew.-% | |
| TAS | 1,9 " | " |
| TS | 0,5 " | " |
| Körnung | | <0,05 mm |
| Einsatzmenge | | 500 g |
| Extraktionsbedingungen : | Druck | 120 bar |
| | Temperatur | 60 ° C |
| | Extraktionsmittel | $CO_2$ |
| Extraktionszeit | | 1 h |

./.

0016899
7. 3. 1979
N 4778/7d

Das Einsatzgut wurde in den als Extraktionsgefäss dienenden Autoklaven so eingebracht, dass ein guter Kontakt zwischen dem überkritischen Gas und der fein gepulverten Rohterephthalsäure gewährleistet war. Während der Extraktionszeit wurde das Extraktionsgefäss langsam von überkritischem $CO_2$ durchströmt. Anschliessend wurde auf unterkritische Bedingungen entspannt und das mit dem $CO_2$ aus dem Autoklaven ausgetragene Produkt nacheinander in 3 Fraktionen abgeschieden. Nach Entfernung des Extraktionsmittels wurde folgende Stoffverteilung und analytische Zusammensetzung festgestellt:

| | g | TPS | TAS | TS |
|---|---|---|---|---|
| Im Extraktionsgefäss verblieben : | 380 | >99,99 % | 35 ppm | <5 ppm |
| 1. Extraktfraktion | 43 | 77 % | 17 % | 6 % |
| 2. Extraktfraktion | 37 | 96 % | 4 % | - |
| 3. Extraktfraktion | 28 | 98,5 % | 1,5 % | - |
| Verlust | 12 | (in den Rohrleitungen verblieben) | | |

Es wurden somit 76 % der eingesetzten Rohterephthalsäure als Terephthalsäure mit einer Verunreinigung von 35 ppm Terephthalaldehydsäure gewonnen.

./.

Patentansprüche :

1. Verfahren zur Gewinnung von reinen Dicarbonsäuren, deren Anhydriden und/oder Estern aus diese Verbindungen enthaltenden Rohprodukten, dadurch gekennzeichnet, dass die Rohprodukte mit Gasen oder Gasgemischen, die sich im überkritischen Zustande befinden, bei Temperaturen oberhalb der kritischen Temperatur und Drücken oberhalb des kritischen Druckes des verwendeten Gases bzw. Gasgemisches einer Extraktion unterworfen werden.

2. Verfahren nach Anspruch 1, dadurch gekennzeichnet, dass als Extraktionsmittel folgende Gase bzw. gasförmigen Verbindungen sowohl in reiner Form als auch in Form von Mischungen untereinander zur Anwendung gelangen :

$CO_2$, $N_2O$, Fluor/Chlor-Kohlenwasserstoffe, $NH_3$, $SO_2$, $CH_3Cl$, $C_2H_4$, $C_2H_6$ und $CH_3OH$.

3. Verfahren nach den Ansprüchen 1 und 2, dadurch gekennzeichnet, dass insbesondere die durch Luftoxidation von Kohlenwasserstoffen gewonnenen Rohrprodukte der Herstellung von Fumarsäure, Maleinsäure, Maleinsäureanhydrid, Phthalsäureanhydrid, Adipinsäure, Adipinsäuredimethylester, Terephthalsäure und Terephthalsäuredimethylester der Extraktion unterworfen werden.

./.

4. Verfahren nach den Ansprüchen 1 bis 3, dadurch gekennzeichnet, dass die zu reinigenden Rohprodukte in Form von Suspensionen oder Lösungen in geeigneten flüssigen Suspensions- oder Lösungsmitteln der Extraktion unterworfen werden.

5. Verfahren nach den Ansprüchen 1 bis 4, dadurch gekennzeichnet, dass die zu reinigenden Rohprodukte in Form von Schmelzen der Extraktion unterworfen werden.

6. Verfahren nach den Ansprüchen 1 bis 5, dadurch gekennzeichnet, dass der Extrakt durch Druck- und/oder Temperaturänderung sowie gegebenenfalls unter Mitverwendung eines Sorptionsmittels aus dem Extraktionsgasstrom abgeschieden wird, wobei die Extraktionsgase nahezu vollständig zurückgewonnen werden.

7. Verfahren nach den Ansprüchen 1 bis 6, dadurch gekennzeichnet, dass die Extraktabscheidung fraktioniert durchgeführt wird.

8. Verfahren nach den Ansprüchen 1 bis 9, dadurch gekennzeichnet, dass der aus den Verunreinigungskomponenten bestehende Extrakt in die der Herstellung des Rohproduktes dienende Verfahrensstufe (Luftoxidation von Kohlenwasserstoff) oder in eine andere vorgeschaltete Verfahrensstufe zurückgeführt wird.

./.

9. Verfahren nach den Ansprüchen 1 bis 8, dadurch gekennzeichnet, dass die Extraktion kontinuierlich oder diskontinuierlich, ein- oder mehrstufig sowie gegebenenfalls unter erneuter Rückführung und erneuter Aufgabe sowohl des Raffinates als auch des Extraktes durchgeführt wird.

10. Verfahren nach den Ansprüchen 1 bis 9, dadurch gekennzeichnet, dass Extraktionsmittel und Einsatzgut im Gegen-, Gleich- oder Kreuzstrom geführt werden.

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 1 390 751 (STUDIEN-GESELLSCHAFT KOHLE) <br><br> * Ansprüche A,B,1,6,9-12,14, 20; Seite 2, Spalte 2, Zeilen 40-48; Seite 3, Spalte 1, Zeilen 30-34; Spalte 2, Zeilen 4-11; Seite 5, Spalte 2, Zeilen 3,4,17-22,33-38; Seite 14, Spalte 2, Zeilen 42-51; Seite 15, Spalte 2, Zeilen 24-36; Seite 16, Spalte 2, Zeilen 41-50; Seite 17, Spalte 1, Zeilen 3-5,35-38 * <br><br> -- | 1-10 |
| A | FR - A - 1 512 060 (THE BRITISH PETROLEUM CO.) <br><br> * Ansprüche 1,5 * <br><br> -- | 1 |
| A | FR - A - 1 512 061 (THE BRITISH PETROLEUM CO.) <br><br> * Ansprüche 1,3 * <br><br> ---- | 1 |

**KLASSIFIKATION DER ANMELDUNG (Int.Cl. 3)**

C 07 C 51/42
        57/145
        51/573
        57/15
C 07 D 307/60
        307/88
C 07 C 55/14
        63/26
        67/48
        69/44
        69/82

**RECHERCHIERTE SACHGEBIETE (Int. Cl. 3)**

C 07 C 51/42
        51/573
        57/145
        55/14
        307/60
        63/26
        307/88
        67/48
        69/44
        69/82
B 01 D 11/02
        11/04

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 18-06-1980 | KLAG |

EPA form 1503.1  06.78